# EUROPEAN PATENT APPLICATION

(11) **EP 2 774 922 A1**
(43) Date of publication of application: **10.09.2014**
(21) Application number: 13157609.2
(22) Date of filing: 04.03.2013
(51) Int. Cl.: C07D 251/70

(54) **Method for obtaining a functionalized triazine compound and the triazine compound obtained by said method**

(71) Applicant: Borealis Agrolinz Melamine GmbH, 4021 Linz (AT)
(72) Inventor: Dicke, Rene, 4060 Leonding (AT)
(74) Representative: Morawski, Birgit

(57) **Abstract**

The present invention relates to a method for obtaining a triazine compound with at least one amino group comprising a an aliphatic moiety with at least two functional groups comprising the steps of
a) providing at least one triazine compound of the general formulae (I) 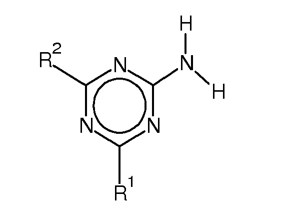 wherein
- R¹ and R² mean independently from each other Q¹ or a moiety of the formula R³-N-R⁴ or R⁵-N-R⁶ bound with its central nitrogen atom to the triazine ring of the structure of formula (I), whereat
- Q¹ means a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl or an amide of a cyclic unsaturated carboxylic acid, whereat in each case one or multiple carbon atoms can be substituted by one or multiple oxygen atoms, sulphur atoms, substituted nitrogen atoms and/or by one or multiple groups of the type -C(O)O-,-OC(O)-,-C(O)- and/or -OC(O)O-,

- R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl,whereat in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O- and/or can be functionalized by one or multiple hydroxyl groups and/or mercapto groups; and

b) reacting said aminotriazine of the general formulae (I) with at least one primary amine of the general formulae (II)

R⁷NH₂

wherein R⁷ is a linear or branched C₃-C₂₀-alkyl, C₅-C₂₀ cycloalkyl, C₅-C₂₀ aryl substituted C₃-C₂₀-alkyl, or C₅-C₂₀-aryl substituted C₅-C₂₀-cycloalkyl, C₅-C₂₀ aryl or C₁-C₂₀ alkylsubstituted C₅-C₂₀ aryl each comprising at least two functional groups, or a mixture of primary amines of the general formulae (II),
wherein the at least two functional groups are not bound to the carbon atom carrying the primary amino group -NH₂, and
wherein the at least two functional groups are not bound to the same carbon atom,

c) wherein the reaction takes place in the presence of at least one acidic catalyst.

The present invention relates also to the compounds obtained by this method and their use.

## Description

The present invention relates to a method for obtaining triazine compounds according to the preamble of claim 1 and triazine compounds obtained by this method according to claim 11.

### Description

Triazine compounds comprising functionalized alkyl moieties bound to an amino group of the triazine are useful comonomers in the synthesis of polymers such as polyesters or formaldehyde based polymers. For instance, melamine derivatives such as N,N',N"-tri-(2-hydroxypropyl) melamine have been shown as being useful for modifying aminoplasts in particular due to their hydroxyl-group providing possibilities for further reactions.

However, so far only triazine compounds and here in particular melamine derivatives are known which comprise preferably only one functional group in an alkyl moiety bound to an amino group restricting the degree of further possible reactions and thus their further use.

In WO2009/121607 A1 a method for obtaining melamine derivatives having at least one amino group substituted with a functionalized moiety is described. Here melamine is reacted with an alcohol which comprises at least one further functional group besides the hydroxyl group. The reaction takes place using a metal or metal oxide as catalyst in an appropriate gas atmosphere.

Another way to obtain a melamine compound with a hydroxyl function is described in EP 178 611 B1. Here a process for the preparation of N,N',N"- tri-(2-hydroxypropyl) melamine is disclosed wherein melamine is reacted with an excess of iso-propanolamine in the presence of an acidic catalyst in a temperature range between 120 and 300°C.

DE 3422218 A1 describes a method for obtaining N,N',N"- tri-(2-hydroxyethyl) melamine comprising the reaction of melamine with ethanolamine in the presence of an acidic catalyst in a temperature range of 120 to 250 °C. N,N',N"- tri-(2-hydroxyethyl) melamine is suitable as a modifier of aminoplast resins used as surface coatings.

The reaction of melamine with either iso-propanolamine or ethanolamine is a transamination reaction wherein a primary amino group of the melamine is replaced by the primary amino group of the iso-propanolamine or ethanolamine.

Transamination reactions are convenient and efficient methods to provide alkylated triazines as shown above.

However, a transamination reaction using amines with more than one additional functional group such as more than one hydroxyl group for instance in form of aminopolyols has so far not been described.

There is therefore a desire to provide a convenient method for synthesizing triazine compounds comprising an aliphatic moiety with more than one functional group.

This object is being solved by providing a method comprising the features of claim 1.

Accordingly, a method for obtaining a triazine compound with at least one amino group comprising a moiety with at least two functional groups is provided which comprises the steps of
a) providing at least one triazine compound of the general formulae (I) 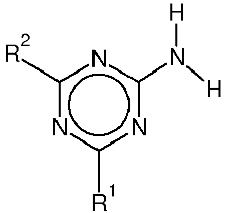 wherein
   - R¹ and R² mean independently from each other Q¹ or a moiety of the formula R³-N-R⁴ or R⁵-N-R⁶ bound with its central nitrogen atom to the triazine ring of the structure of formula (I), whereat
      - Q¹ means a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl or an amide of a cyclic unsaturated carboxylic acid, whereat in each case one or multiple carbon atoms can be substituted by one or multiple oxygen atoms, sulphur atoms, substituted nitrogen atoms and/or by one or multiple groups of the type -C(O)O-,-OC(O)-,-C(O)- and/or -OC(O)O-,
   - R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, which in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O- and/or can be functionalized by one or multiple hydroxyl groups and/or mercapto groups; or an amide of a carboxylic acid or an imide of a cyclic dicarboxylic acid, and
b) reacting said aminotriazine of the general formulae (I) with at least one primary amine of the general formulae (II)

   R⁷NH₂

   wherein R⁷ is a linear or branched C₃-C₂₀-alkyl, C₅-C₂₀-cycloalkyl, C₅-C₂₀-aryl substituted C₃-C₂₀-alkyl, or C₅-C₂₀-aryl substituted C₅-C₂₀-cycloalkyl, C₅-C₂₀ aryl or C₁-C₂₀ alkylsubstituted C₅-C₂₀ aryl each comprising at least two functional groups, or a mixture of primary amines of the general formulae (II),
   wherein the at least two functional groups are not bound to the carbon atom carrying the primary amino group -NH₂, and
   wherein the at least two functional groups are not bound to the same carbon atom,
c) wherein the reaction takes place in the presence of at least one acidic catalyst.

The present method allows for the specific modification of a primary amino group of a triazine by transamination using a second primary amine of the general formulae (II). The present method allows specifically for the transamination using a primary amine with at least two functional groups in the molecule (besides the primary amino group). Furthermore, when using a mixture of primary amine of the general formulae (II) it is also possible to obtain mixed melamine structure since different R⁷ moieties may be introduced into the aminotriazine.

Thus, a general method is provided wherein polyfunctional moieties can be introduced into the triazine molecule in an easy manner without the need for any cumbersome protection groups as usually applied using functional groups.

The at least two functional groups present in R₇ can also be described as electron withdrawing groups. In an electron withdrawing group, in accordance with at least one embodiment of the invention, groups and substituents are understood to portray a negative inductive effect (that is, -I-effect) and thereby reduce the electron density in the local environment.

As stated above the functional groups are not bound to the carbon atom carrying the primary amino group -NH₂. This means that a functional group is not bound to the C1 atom of R⁷, but rather to C2, C3 and even in a further distance to the amino group of R⁷NH₂. Thus, the distance between the primary amino group and a first functional group comprises preferably 2 C atoms, such as in -CH₂-CH₂-. It is however also possible that a first functional group is placed even further away such as with a distance of 3, 4 or 5 C-atoms. The distance of a functional group from the amino group may influence the overall reactivity of the amino group.

As stated also above the at least two functional groups are not bound to the same carbon atom in the alkyl moiety R⁷. For instance, the use of a ketal or acetal or aminal as a functional group is thus not possible and is exempted.

It is preferred if the R¹ and/or R² are a moiety of the formula R³-N-R⁴ or R⁵-N-R⁶ bound with its central nitrogen atom to the triazine ring of the structure of formula (I), wherein R³, R⁴, R⁵ and R⁶ mean independently from each other H, linear or branched C₁-C₁₀-alkyl (such as methyl, ethyl, propyl, butyl) or C₅-C₁₀-cyclo alkyl (such a cyclopentyl, cyclohexyl), whereat in each case one or multiple carbon atoms can be substituted by one or multiple oxygen atoms, wherein the oxygen atom has at least a distance of (-CH₂-)ₙ with n≥2, such as (-CH₂-CH₂-) from the N-atom, such as, ethylenglycol, diethylenglycol or morpholine (in case of morpholine the N-atom of the morpholine is the N-atom of the exocyclic amino group of the triazine), or can be functionalised with hydroxyl or mercapto groups, such as hydroxyethyl, mercaptoethyl. Thus, the N-atom may be substituted by an ether type moiety.

In a more preferred embodiment of the present method the triazine used in step a) is preferably of the general formulae (Ia) 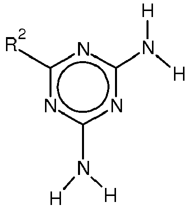 wherein R² has the above meaning. Thus, it may be preferred to use a triazine comprising at least two primary amino groups.

In the case of the compound of formulae (Ia) R¹ is a moiety of the formula R³-N-R⁴ bound with its central nitrogen atom to the triazine ring of the structure of formula (I), wherein R³ and R⁴ mean H.

In a most preferred embodiment of the present method melamine is used as triazine in step a). In this case R¹ and/or R² are a moiety of the formula R³-N-R⁴ or R⁵-N-R⁶ bound with its central nitrogen atom to the triazine ring of the structure of formula (I), wherein R³, R⁴, R⁵ and R⁶ mean H.

In an embodiment of the present method the transamination reaction is carried out in a suitable solvent or solvent free environment. The term "solvent" within the meaning of the present method means thereby a substance which is able to dissolve or dilute a compound without undergoing a chemical reaction between the solvent and the compound to be dissolved. Thus, the present method does not require such a solvent. If the reaction is carried out in a suitable solvent, in particular organic solvent may be selected from a group comprising tetrahydrofuran (THF), dioxane, glycol-dimethylether, ethylenglycol, diethylenglycol, alcohols, like ethanol, propanol or butanol, dimethylacetamide, dimethylformamide.

In a further embodiment of the present method the moiety R⁷ of the at least one primary amine of the general formulae (II) is selected from a group comprising linear or branched C₃-C₁₀-alkyl or C₅-C₁₀-cycloalkyl, C₆-C₁₄ aryl, C₁-C₁₀ alkysubstituted C₆-C₁₄ aryl or C₆-C₁₄ aryl substituted C₃-C₁₀-alkyl or C₆-C₁₄ aryl substituted C₅-C₁₀-cycloalkyl each comprising at least two functional groups. R⁷ is preferably n-propyl, isopropyl, n-butyl, s-butyl, t-butyl, isobutyl, pentyl, cyclopentyl, hexyl, cyclohexyl, 2-ethyl-hexyl, octyl, decyl, phenyl-propyl, phenyl-butyl each comprising at least two functional groups. R⁷ is preferably 3-amino-2-hydroxy-propyl, 1,3-dihydroxy-iso-propyl, di-1,1-hydroxymethyl-ethyl, 1,3-dihydroxy-l-phenyl-iso-propyl, di-1,1-hydroxymethyl-propyl, tris-hydroxymethyl-methyl, 2,3-dihydroxy-propyl.

It is also preferred if the at least two functional groups are selected from a group comprising OH; amine; thiol and alkoxy. In particular preferred are OH, primary and/or secondary amino groups. The at least two functional groups can be the same or different.

In a most preferred embodiment of the present method the primary amine of formula (II) is selected from group comprising 3-amino-1,2-propandiol such as (S)-3-amino-1,2-propanediol, (R)-3-amino-1,2-propanediol, (R,S)-3-amino-1,2-propanediol, 2-amino-1,3-propandiol, 2-amino-2-methyl-1,3-propandiol, 4-amino-4-(3-hydroxypropyl)-1,7-heptanediol, 2-amino-1-phenyl-1,3-propanediol, 2-amino-2-ethyl-1,3-propanediol, 4-(2-amino-1-hydroxyethyl)-benzene-1 ,2-diol, 1,3-diamino-2-propanol, N-(2-hydroxyethyl)ethylenediamine,

In an embodiment of the present method the acidic catalyst is an organic or inorganic acid, in particular H₂SO₄, H₃PO₄, HCI, HNO₃, formic acid, acetid acid, toluene sulfonic acid, ion exchange resins, acid activated alumosilicates and any other suitable acid.

The acidic catalyst is preferably used in an amount between 1 and 50 mol%, preferably 10 and 20 mol% in respect to the triazine.

In a variant of the present method the ratio of the triazine of formulae (I) and the primary amine of formulae (II) is 1:50, preferably 1:20, most preferably 1:10. In general a higher amount of primary amine increases the reaction velocity.

The present transamination reaction is preferably carried out at a temperature in a range between 150 and 250 °C, preferably 180 and 220 °C. In general the conversion rate of the triazine increases with temperature, i.e. the higher the temperature the higher the yield of the transaminated product. This is most probably due to an increased solubility of the triazine used as starting compound in the reaction mixture.

It is also preferred if the present reaction is carried out for at least 2 h up to 72 h, preferably 24 h to 48 h. It is furthermore preferred if a the reaction is carried out using a temperature cascade which starts at 220 °C for 3 h to 6 h, followed at 180 °C for 40 h to 72 h, preferably 50 h to 66 h.

It is also preferred, if the transamination reaction is carried out in an inert gas atmosphere such as in the presence of helium, nitrogen, argon or in the presence of a gas which does not undergo any reaction at the described conditions such as ammonia. This reduces or even avoids side reactions.

It is also possible and preferred if after the reaction is completed the obtained product mixture is at first neutralised before any further work up steps are carried out. Neutralisation can be done by adding any suitable base such as NaOH or KOH.

The raw product, preferably obtained after neutralisation, undergoes further work up such as recrystallisation or extraction using as solvents water, or alcohols, such as ethanol, butanol, isopropanol, or ketons, such as acetone or MIBK, or cyclic ethers, such as THF or dioxane, or mixtures thereof. The work up can be for instance carried out in such a way that the reaction mixture is heated up under reduced pressure to remove the solvent and the excess of the at least one primary amine of the general formulae (II). The raw product is then heated and dissolved in a suitable solvent and the suspension is hot filtrated. The filtrate is subsequently cooled and the crystallized product is separated and analyzed.

The reaction mechanism of the present transamination process is now exemplarily shown using melamine as triazine and 3-amino-1,2-propandiol as primary amine:

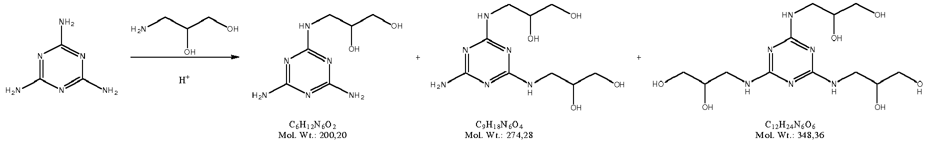

In above reaction scheme the primary amino groups of melamine undergo a stepwise transamination reaction with 3-amino-1,2-propandiol. The above reaction setup for 3-amino-1,2-propandiol provides a selectivity of more than 99% for the three times substituted product.

It is to be understood that above reaction is only one possible example and that the present invention is not restricted to these specific compounds but that rather multiple different triazines and primary amines may be used as described in detail above.

It is also to be noted that when using a primary amine of general formulae (II) having a terminal amino group then preferably a three times transamination on the amino groups of the triazine occurs (i.e. each of the three amino groups of a triazine such as melamine comprises at least one moiety R⁷), whereas when using a primary amine of general formulae (II) wherein the amino group is for instance bound to a branched (i.e. secondary or tertiary) C-Atom, such as a -CHRR' or -CRR'R" then preferably a single or double transamination occurs (i.e. only one or two of the three amino groups of a triazine such as melamine comprises at least one moiety R⁷). Thus, the triazine reacts once or twice with the primary amine of formulae (II). As mentioned previously, the distance of the functional group from the amino group in the primary amine of formulae (II) also influences selectivity and yield of the reaction.

When applying the reactions conditions as described above at least one compound of the general formulae (IIIa) 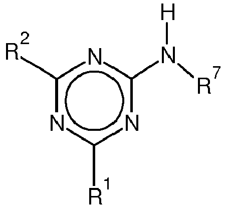 is obtained, wherein R¹, R² and R⁷ have the above meanings.

When using a triazine compound of general formulae (Ia) then preferably a compound of the general formulae (IIIb) 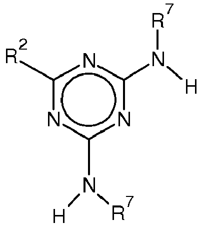 is obtained wherein R² and R⁷ have the above meanings, and wherein R⁷ can be the same or different.

When using melamine as triazine a compound of the general formulae (IIIc) 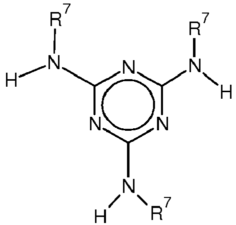 is obtained, wherein R⁷ has the above meanings.

Therefore, the object of the present invention is also being solved by providing at least one of the compounds of the general formulae (IIIa), (IIIb) and/or (IIIc), whereat preferably N-mono-(2,3-dihydroxypropyl)melamine, N,N'-di-(2,3-dihydroxypropyl)melamine, N,N',N"-tris-(2,3-dihydroxypropyl)melamine are exempted from the above class of compounds, respectively.

As described previously the moiety R⁷ is selected from a group comprising linear or branched C₃-C₁₀-alkyl or C₅-C₁₀-cycloalkyl, C₆-C₁₄ aryl, C₁-C₁₀ alkysubstituted C₆-C₁₄ aryl or C₆-C₁₄ aryl substituted C₃-C₁₀-alkyl or C₆-C₁₄ aryl substituted C₅-C₁₀-cycloalkyl each comprising at least two functional groups. R⁷ is preferably n-propyl, isopropyl, n-butyl, s-butyl, t-butyl, isobutyl, pentyl, cyclopentyl, hexyl, cyclohexyl, 2-ethyl-hexyl, octyl, decyl, phenyl-propyl, phenyl-butyl each comprising at least two functional groups. R⁷ is preferably 3-amino-2-hydroxy-propyl, 1,3-dihydroxy-iso-propyl, di-1,1-hydroxymethyl-ethyl, 1,3-dihydroxy-1-phenyl-iso-propyl, di-1,1-hydroxymethyl-propyl, tris-hydroxymethyl-methyl, 2,3-dihydroxy-propyl.

R⁷ thus does not comprise ether, thioether, or tertiary amino group, carbonyl, -C(O)O- and/or -OC(O)O- group within its moiety in addition to the at least tow functional groups, respectively. For instance, moieties like N,N-di-(2-hydroxyethyl)-2- aminoethyl or N,N-di-(2-hydroxy-iso-propyl)-3-amino-isopropyl may be exempted.

It is in particular preferred that the compound obtained by the present method is selected from a group comprising
- N-mono-(1,3-dihydroxy-iso-propyl) melamine, N,N'-di-(1,3-dihydroxy-isopropyl) melamine, N,N',N"-tris-(1,3-dihydroxy-iso-propyl) melamine,
- N-mono-(di-1,1-hydroxymethyl-ethyl) melamine, N,N'-di-(di-1,1-hydroxymethyl-ethyl) melamine, N,N',N"-tri-(di-1,1-hydroxymethyl-ethyl) melamine,
- N-mono-(tris-hydroxymethyl-methyl) melamine, N,N'-di-(tris-hydroxymethyl-methyl) melamine, N,N',N"-tri-(tris-hydroxymethyl-methyl) melamine,
- N-mono-(1,3-dihydroxy-1-phenyl-iso-propyl) melamine, N,N'-di-(1,3-dihydroxy-1-phenyl-iso-propyl) melamine, N,N',N"-tris-(1,3-dihydroxy-1-phenyl-iso-propyl) melamine,
- N-mono-(di-1,1-hydroxymethyl-propyl) melamine, N,N'-di-(di-1,1-hydroxymethyl-propyl) melamine, N,N',N"-tri-(di-1,1-hydroxymethyl-propyl) melamine,
- N-mono-(3-amino-2-hydroxy-propyl) melamine, N,N'-di-(3-amino-2-hydroxypropyl) melamine, N,N',N"-tris-(3-amino-2-hydroxy-propyl) melamine,
- N-mono-(2,3-dihydroxy-propyl)-acetoguanamine, N,N'-di-(2,3-dihydroxy-propyl) acetoguanamine,
- N-mono-(2,3-dihydroxy-propyl)-benzoguanamine, N,N'-di-(2,3-dihydroxy-propyl) benzoguanamine,
- N-mono-(2,3-dihydroxy-propyl)-morpholino-melamine, N,N'-di-(2,3-dihydroxy-propyl) morpholino-melamine.

The chemical structures of the listed compounds are shown in the following for a better understanding:

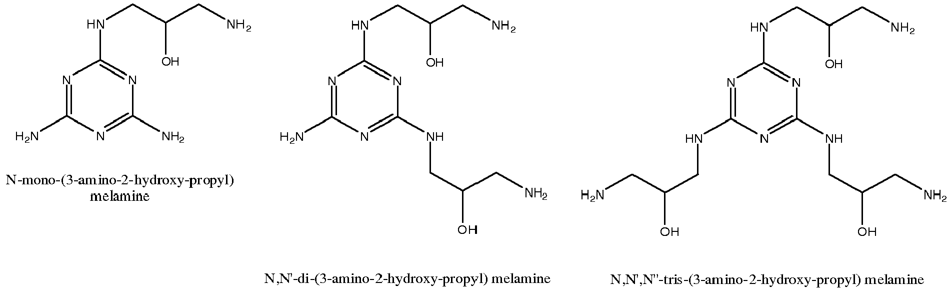

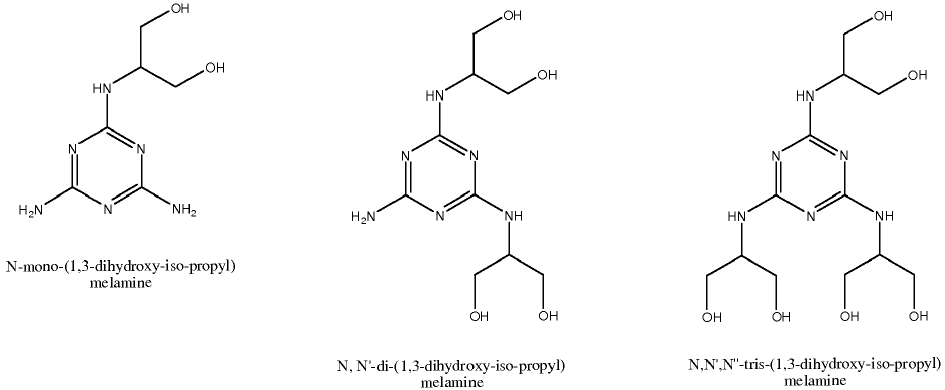

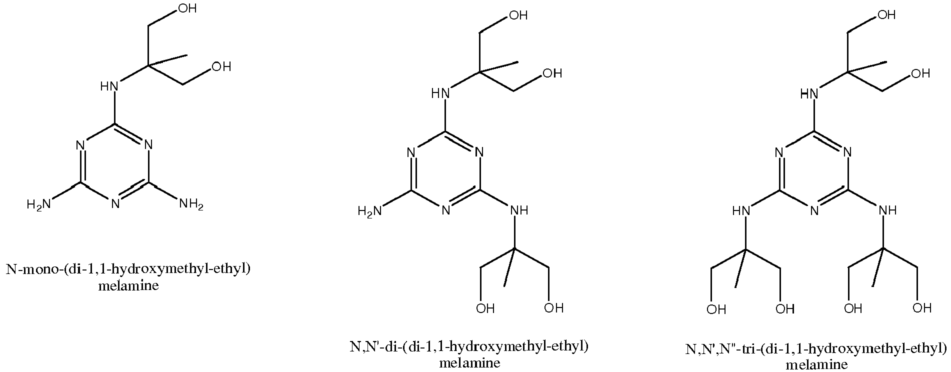

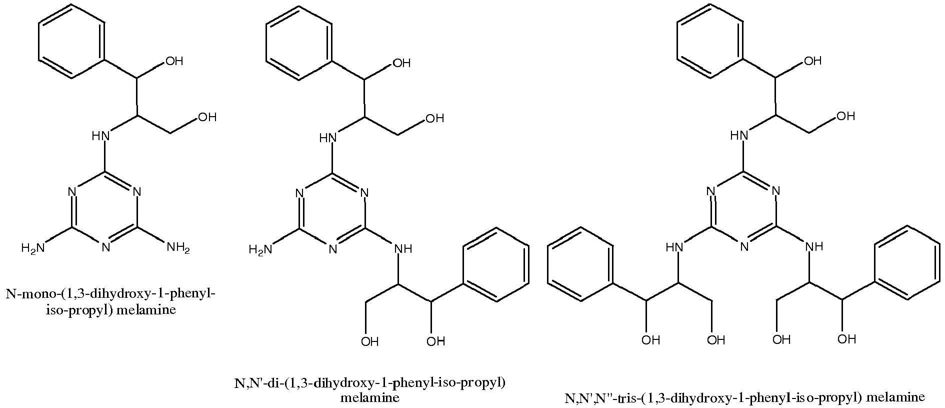

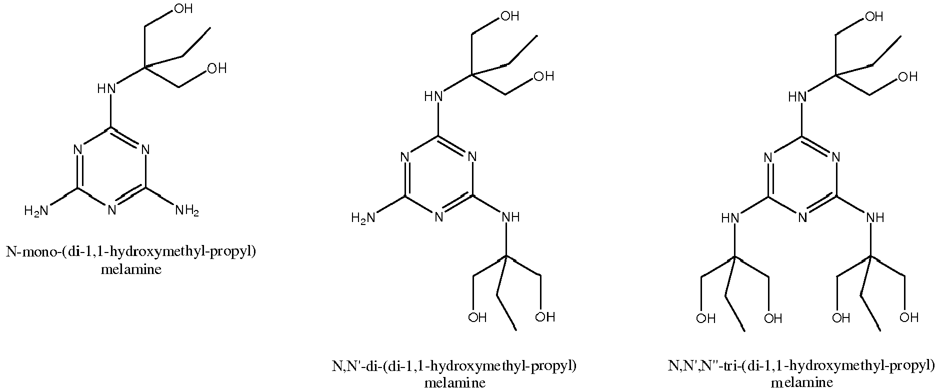

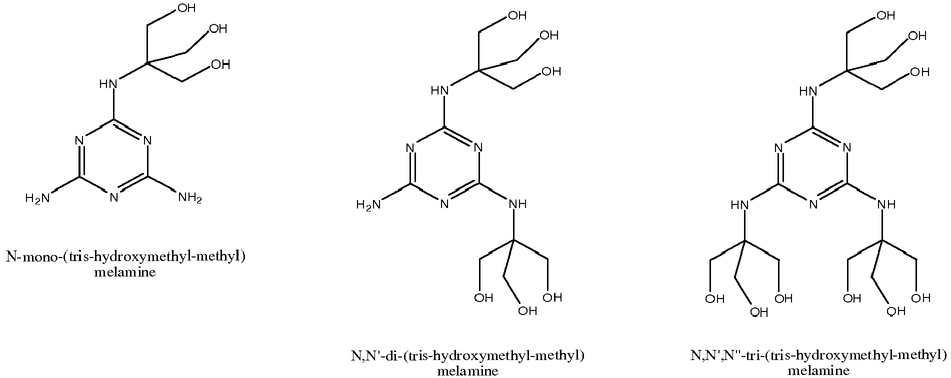

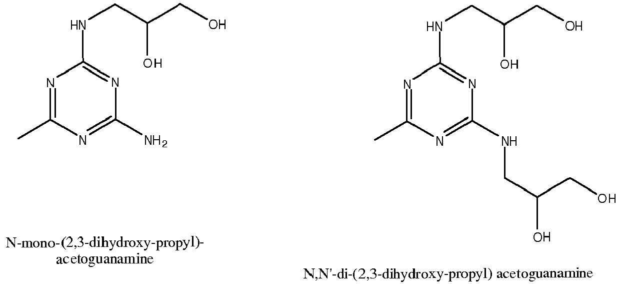

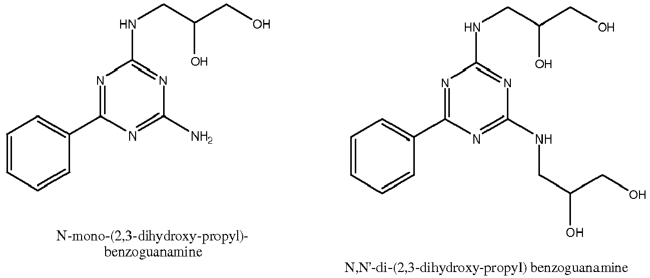

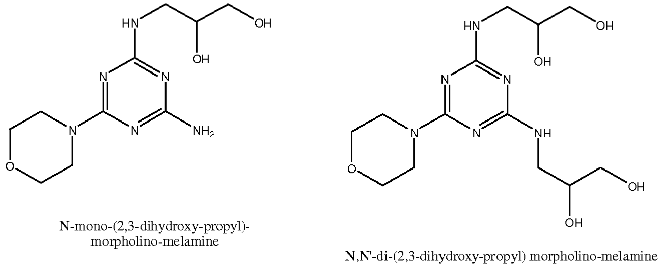

The compounds obtained by the present method are in particular useful in the synthesis of polymers, for instance as additives.

Further details of the invention will be explained by the means of the following examples.

### Example 1: Transamination of melamine with 3-amino-1,2-propandiol

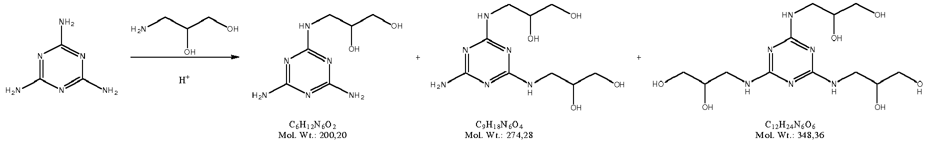

A mixture of melamine (1.26 g, 10 mmol), 3-amino-1,2-propandiol (9.1 g, 100 mmol) and concentrated sulfuric acid (200 mg, 2mmol, 20 mol%) are stirred for 72 hours at 180° C in an argon atmosphere. A sample is taken after 40 hours and 48 hours. The final product and both samples are analyzed by HPLC. The yield calculated by HPLC-quantification is 89 %.

HPLC-analysis provides the following results:

After 40 hours: less than 0.1% melamine, 6.4 % N-mono-(2,3-dihydroxypropyl)melamine (DHPM1), 32.4 % N,N'-di-(2,3-dihydroxypropyl)melamine (DHPM2), and 61.1 % N,N',N"-tris-(2,3-dihydroxypropyl)melamine (DHPM3).

After 48 hours: less than 0.1% melamine, 3.6 % DHPM1, 26.8 % DHPM2, and 69.5 % DHPM3.

After 72 hours: less than 0.1% melamine, less than 0.1 % DHPM1, 11.4 % DHPM2, and 88.5 % DHPM3.

### Example 2: A further transamination of melamine with 3-amino-1,2-propandiol

A mixture of melamine (1.26 g, 10 mmol), 3-amino-1,2-propanediol (9.1 g, 100 mmol), and concentrated sulfuric acid (200 mg, 2mmol, 20 mol%) are stirred at first for 6 hours at 220° C and then 66 hours at 180° C. A sample is taken after 27 hours and 48 hours. The final product and both samples are analyzed by HPLC. The overall yield calculated by HPLC-quantification is 87 %.

The analysis provides the following results:

After 27 hours: less than 0.1% melamine, 0.2 % DHPM1, 2.8 % DHPM2, and 96.9 % DHPM3.

After 48 hours: less than 0.1% melamine, less than 0.1% DHPM1, 0.8% DHPM2, and 99.0% DHPM3.

After 72 hours: 0.3 % DHPM2, 99.7 DHPM3.

### Example 3: Transamination of melamine with 2-amino-1,3-propandiol

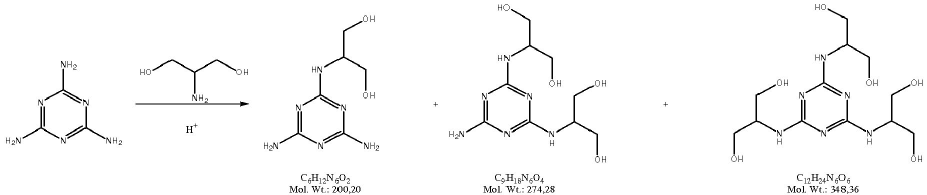

A mixture of melamine (1.26 g, 10 mmol), 2-amino-1,3-propandiol (9.1 g, 100 mmol) and concentrated sulfuric acid (100 mg, 1 mmol, 10 mol%) is stirred for 48 hours at 180° C under an argon atmosphere. A sample is taken after 24 hours and 48 hours. The final product and the sample are analyzed by HPLC. The yield calculated by HPLC-quantification is 78.5%.

The analysis provides the following results:

After 24 hours: 10.2% melamine, 79.2 % N-mono-(1,3-dihydroxy-iso-propyl)melamine (1,3-DHIPM1) 10.5 % N,N'-di(1,3-dihydroxy-iso-propyl)melamine (1,3-DHIPM2), and less than 0.1 % N,N',N"-tris(1,3-dihydroxy-iso-propyl)melamine (1,3-DHIPM3).

After 48 hours: 4.8% melamine, 69.5 % 1,3- DHIPM1, 24.7 % 1,3- DHIPM2, and 3.1 % 1,3-DHIPM3.

### Example 4: Transamination of melamine with 2-amino-2-methyl-1,3-propandiol

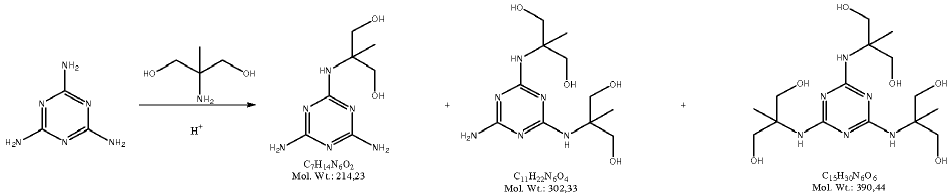

The mixture of melamine (1.26 g, 10 mmol), 2-amino-2-methyl-1,3-propanediol (10.5 g, 100 mmol), and concentrated sulfuric acid (500 mg, 51 mmol, 50 mol%) is stirred for 24 hours at 200° C under an argon atmosphere. The product is analyzed by HPLC. The yield calculated by HPLC-quantification is 69 %.

After 24 hours: 22.3% melamine, 68.4 % N-mono-(1,1-dihydroxymethyl-ethyl)melamine (1,1-DHMEM1) 8.9 % N,N'-di(1,1-dihydroxymethyl-ethyl)melamine (1,1-DHMEM2), and less than 0.1 % N,N',N"-tris(1,1-dihydroxymethyl-ethyl)melamine (1,1-DHMEM3).

### Example 5: Transamination of melamine with trishydroxymethylaminomethane

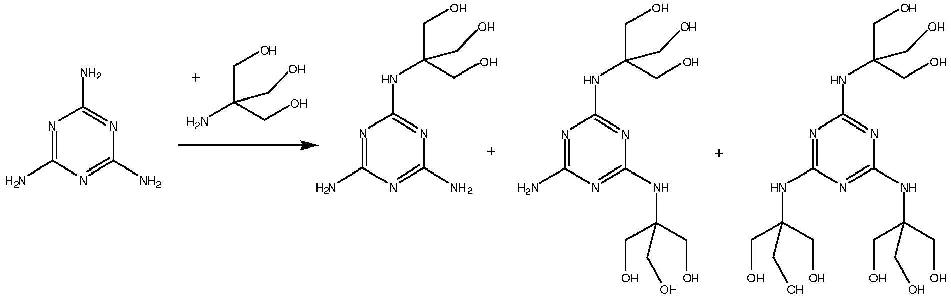

A mixture of melamine (1.26 g, 10 mmol), trishydroxymethylaminomethane (12.1 g, 100 mmol) and concentrated sulfuric acid (200 mg, 2mmol, 20 mol%) are stirred for 72 hours at 180° C in an argon atmosphere. The final product mixture is analyzed by HPLC. The yield calculated by HPLC-quantification is 73 %.

### HPLC-analysis provides the following results:

5% melamine, 15.5 % N-mono-(trihydromethyl-methyl)melamine (THMMM1), 55.8 % N,N'-di-( trihydromethyl-methyl)melamine (THMMM2), and 18.4 % N,N',N"-tris-( trihydromethyl-methyl)melamine (THMMM3).

### Example 6: Transamination of melamine with 1,3-diamino-2-propanol

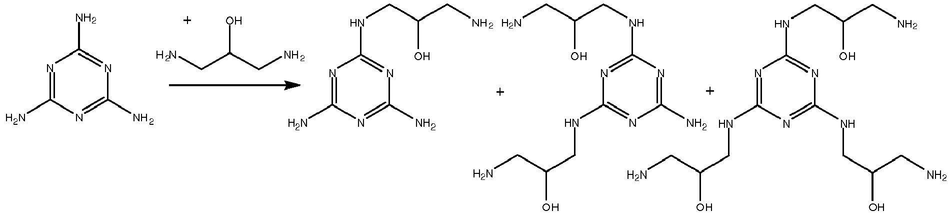

A mixture of melamine (1.26 g, 10 mmol), 1,3-diamino-2-propanol (9.01 g, 100 mmol) and concentrated sulfuric acid (200 mg, 2mmol, 20 mol%) are stirred for 72 hours at 180° C in an argon atmosphere. The final product mixture is analyzed by HPLC. The yield calculated by HPLC-quantification is 82 %.

### HPLC-analysis provides the following results:

1.2% melamine, 7.3% N-mono-(3-amino-2-hydroxy-propyl) melamine (AHPM1), 32.7% N,N'-di-(3-amino-2-hydroxy-propyl) melamine (AHPM2), 52.5% N,N',N"-tris-(3-amino-2-hydroxypropyl) melamine (AHPM3), and 6.3% oligomeric melamine derivatives.

## Claims

1. Method for obtaining a triazine compound with at least one amino group comprising a moiety with at least two functional groups
comprising the steps of
a) providing at least one triazine compound of the general formulae (I) 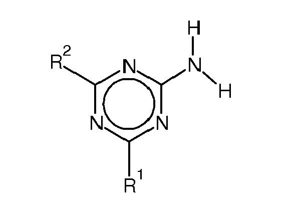 wherein
- R¹ and R² mean independently from each other Q¹ or a moiety of the formula R³-N-R⁴ or R⁵-N-R⁶ bound with its central nitrogen atom to the triazine ring of the structure of formula (I), whereat
- Q¹ means a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl or an amide of a cyclic unsaturated carboxylic acid, whereat in each case one or multiple carbon atoms can be substituted by one or multiple oxygen atoms, sulphur atoms, substituted nitrogen atoms and/or by one or multiple groups of the type -C(O)O-,-OC(O)-,-C(O)- and/or -OC(O)O-,
- R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl,whereat in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O- and/or can be functionalized by one or multiple hydroxyl groups and/or mercapto groups; and
b) reacting said aminotriazine of the general formulae (I) with at least one primary amine of the general formulae (II)
R⁷NH₂
wherein R⁷ is a linear or branched C₃-C₂₀-alkyl, C₅-C₂₀-cycloalkyl, C₅-C₂₀-aryl substituted C₃-C₂₀-alkyl, C₅-C₂₀-aryl substituted C₅-C₂₀-cycloalkyl, C₅-C₂₀-aryl or C₁-C₂₀-alkylsubstituted C₅-C₂₀-aryl each comprising at least two functional groups, or a mixture of primary amines of the general formulae (II),
wherein the at least two functional groups are not bound to the carbon atom carrying the primary amino group -NH₂, and
wherein the at least two functional groups are not bound to the same carbon atom,
c) wherein the reaction takes place in the presence of at least one acidic catalyst.

2. Method according to claim 1, **characterized in that** R⁷ is selected from a group comprising linear or branched C₃-C₁₀-alkyl, C₅-C₁₀ cycloalkyl, C₆-C₁₄ aryl or C₁-C₁₀ alkylsubstituted C₆-C₁₄ aryl or C₆-C₁₄ aryl substituted C₃-C₁₀-alkyl or C₆-C₁₄ aryl substituted C₅-C₁₀-cycloalkyl each comprising at least two functional groups.

3. Method according to claim 1 or 2, **characterized in that** R⁷ is selected from a group comprising n-propyl, isopropyl, n-butyl, s-butyl, t-butyl, isobutyl, pentyl, cyclopentyl, hexyl, cyclohexyl, 2-ethyl-hexyl, octyl, decyl, phenyl-propyl, phenyl-butyl each comprising at least two functional groups

4. Method according to one of the preceding claims, **characterized in that** the at least two functional groups in R⁷ are the same or different from each other.

5. Method according to one of the preceding claims, **characterized in that** the at least two functional groups in R⁷ are selected from a group comprising OH; amine; thiol and alkoxy.

6. Method according to one of the preceding claims, **characterized in that** the at least one primary amine of the general formulae (II) is selected from a group comprising 3-amino-1,2-propandiol, 2-amino-1,3-propandiol, 2-amino-2-methyl-1,3-propandiol, 4-Amino-4-(3-hydroxypropyl)-1,7-heptanediol, 2-Amino-1-phenyl-1,3-propanediol, 2-Amino-2-ethyl-1,3-propanediol, 4-(2-Amino-1-hydroxy-ethyl)-benzene-1,2-diol,1,3-Diamino-2-propanol, N-(2-Hydroxyethyl)ethylenediamine.

7. Method according to one of the preceding claims, **characterized in that** the at least one acid is an organic or inorganic acid, in particular H₂SO₄, H₃PO₄, HCI, HNO₃, formic acid, acetid acid, toluene sulfonic acid, ion exchange resins, acid activated alumosilicates.

8. Method according to one of the preceding claims, **characterized in that** the ratio of the triazine of formulae (I) and the primary amine of formulae (II) is 1 : 50, preferably 1:20, most preferably 1:10.

9. Method according to one of the preceding claims, **characterized in that** the triazine used in step a) is of the general formulae (Ia) 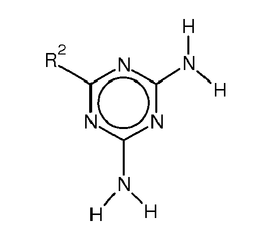 wherein R² has the above meaning.

10. Method according to one of the preceding claims, **characterized in that** melamine is used as triazine in step a).

11. Compound obtained by a method according to one of the preceding claims **characterized by** the general formulae (IIIa) 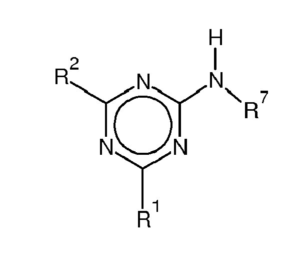 wherein R¹, R² and R⁷ have the above meanings, wherein R⁷ can be the same or different and whereat N-mono-(2,3-dihydroxypropyl)melamine, N,N'-di-(2,3-dihydroxypropyl)melamine, N,N',N"-tris-(2,3-dihydroxypropyl)melamine are exempted.

12. Compound according to claim 11 **characterized by** the general formulae (IIIb) 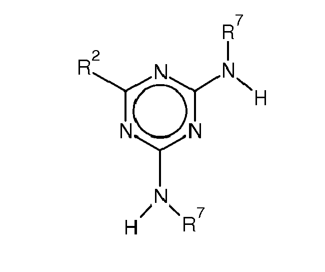 wherein R² and R⁷ have the above meanings.

13. Compound according to claim 11 or 12 **characterized by** the general formulae (IIIc) 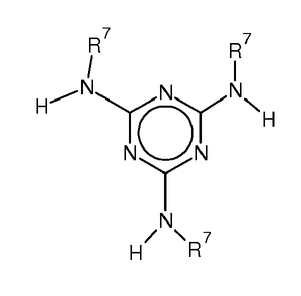 wherein R⁷ has the above meanings.

14. Compound according to one of the claims 11 to 13, **characterized in that** the compound is selected from the group comprising
- N-mono-(1,3-dihydroxy-iso-propyl) melamine, N,N'-di-(1,3-dihydroxy-iso-propyl) melamine, N,N',N"-tris-(1,3-dihydroxy-iso-propyl) melamine,
- N-mono-(di-1,1-hydroxymethyl-ethyl) melamine, N,N'-di-(di-1,1-hydroxymethyl-ethyl) melamine, N,N',N"-tri-(di-1,1-hydroxymethyl-ethyl) melamine,
- N-mono-(tris-hydroxymethyl-methyl) melamine, N,N'-di-(tris-hydroxymethyl-methyl) melamine, N,N',N"-tri-(tris-hydroxymethyl-methyl) melamine,
- N-mono-(1,3-dihydroxy-1-phenyl-iso-propyl) melamine, N,N'-di-(1,3-dihydroxy-1-phenyl-iso-propyl) melamine, N,N',N"-tris-(1,3-dihydroxy-1-phenyl-iso-propyl) melamine,
- N-mono-(di-1,1-hydroxymethyl-propyl) melamine, N,N'-di-(di-1,1-hydroxymethyl-propyl) melamine, N,N',N"-tri-(di-1,1-hydroxymethyl-propyl) melamine,
- N-mono-(3-amino-2-hydroxy-propyl) melamine, N,N'-di-(3-amino-2-hydroxypropyl) melamine, N,N',N"-tris-(3-amino-2-hydroxy-propyl) melamine,
- N-mono-(2,3-dihydroxy-propyl)-acetoguanamine, N,N'-di-(2,3-dihydroxy-propyl) acetoguanamine,
- N-mono-(2,3-dihydroxy-propyl)-benzoguanamine, N,N'-di-(2,3-dihydroxy-propyl) benzoguanamine, and
- N-mono-(2,3-dihydroxy-propyl)-morpholino-melamine, N,N'-di-(2,3-dihydroxy-propyl) morpholino-melamine.
